# EUROPEAN PATENT APPLICATION

(11) **EP 4 410 955 A1**
(43) Date of publication of application: **07.08.2024**
(21) Application number: 22876375.1
(22) Date of filing: 28.09.2022
(51) Int. Cl.: C12N 1/21, C12P 7/625, C12N 15/31, C12N 15/53

(54) **METHOD FOR CULTURING MICROORGANISM, TRANSFORMED MICROORGANISM, AND PRODUCTION METHOD OF POLY(3-HYDROXYALKANOATE)**

(30) Priority: 30.09.2021 JP 2021160749
(71) Applicant: Kaneka Corporation, Osaka-shi, Osaka 530-8288 (JP)
(72) Inventor: HARADA, Ken, Takasago-shi, Hyogo 676-8688 (JP); SATO, Shunsuke, Takasago-shi, Hyogo 676-8688 (JP); ARIKAWA, Hisashi, Takasago-shi, Hyogo 676-8688 (JP)
(74) Representative: Vossius & Partner Patentanwälte Rechtsanwälte mbB
(86) International application number: PCT/JP2022/036257
(87) International publication number: WO 2023/054509

(57) **Abstract**

A microorganism is cultivated in the presence of a carbon source. The microorganism is a transformed microorganism having an introduced fadH gene encoding 2,4-dienoyl-CoA reductase. That is, the transformed microorganism has an introduced foreign gene encoding a protein having an amino acid sequence of SEQ ID NO: 1 or an introduced foreign gene encoding a protein having an amino acid sequence that is at least 85% identical to the amino acid sequence of SEQ ID NO: 1. The carbon source contains an oil containing unsaturated fatty acids as constituents or free unsaturated fatty acids.

## Description

### Technical Field

The present invention relates to a method for culturing a microorganism, a transformed microorganism, and a method for producing a poly(3-hydroxyalkanoate).

### Background Art

Techniques for culturing microorganisms and exploiting the microorganisms to produce useful substances by fermentation are in widespread industrial use. Known examples of the useful substances include poly(3-hydroxyalkanoates) (also referred to as "PHAs" hereinafter). PHAs are used as plastic materials and, in recent years, have attracted attention as environmentally benign materials since they are biodegradable after use.

For culturing a microorganism to produce a useful substance such as a PHA, it is necessary to supply a carbon source that is suitably assimilated by the microorganism. Typical examples of the carbon source include sugars, oils, and free fatty acids.

There are many examples where fermentative production is carried out using an oil as a carbon source. For example, Patent Literature 1 describes PHA production in which a microorganism having a PHA-producing ability is cultured using a palm oil as a carbon source.

### Citation List

### Patent Literature

PTL 1: Japanese Laid-Open Patent Application Publication (Translation of PCT Application) No. 2013-510572
PTL 2: WO 2014/061804

### Summary of Invention

### Technical Problem

The present inventors attempted microbial culture using various oils as carbon sources. As a result, the present inventors have discovered that slow proliferation rate and low PHA productivity are observed when the microorganism is cultivated in culture where an oil having a relatively high iodine value, namely an oil containing a relatively large amount of unsaturated fatty acids as constituents (e.g., rapeseed oil), or a free unsaturated fatty acid as a carbon source.

In view of the above circumstances, one aspect of the present invention aims to provide a method for culturing a microorganism, the method being adapted to achieve a high cell proliferation rate despite the use of an oil containing unsaturated fatty acids as constituents or of a free unsaturated fatty acid as a carbon source.

Other aspects of the present invention aim to provide a transformed microorganism and a method for producing a PHA, the transformed microorganism and the method being adapted to achieve a high cell proliferation rate and a high PHA production rate despite the use of an oil containing unsaturated fatty acids as constituents or of a free unsaturated fatty acid as a carbon source.

### Solution to Problem

The present inventors have investigated for introducing a foreign gene involved in unsaturated fatty acid metabolism into a microorganism, with the goal of enabling the microorganism to efficiently assimilate an oil as a carbon source which contains a relatively large amount of unsaturated fatty acids such as rapeseed oil. The present inventors have found that introducing a fadH gene encoding 2,4-dienoyl-CoA reductase as the foreign gene results in an increase in cell proliferation rate and an increase in PHA production rate. Based on this finding, the inventors have arrived at the present invention.

The present invention relates to a method for culturing a microorganism, the method including: culturing the microorganism in the presence of a carbon source, wherein the microorganism is a transformed microorganism having an introduced foreign gene encoding a protein having an amino acid sequence of SEQ ID NO: 1 or an introduced foreign gene encoding a protein having an amino acid sequence that is at least 85% identical to the amino acid sequence of SEQ ID NO: 1, and the carbon source contains an oil containing unsaturated fatty acids as constituents or contains free unsaturated fatty acids.

The present invention further relates to a transformed microorganism having: a gene encoding a poly(3-hydroxyalkanoate) synthase; and an introduced foreign gene encoding a protein having an amino acid sequence of SEQ ID NO: 1 or an introduced foreign gene encoding a protein having an amino acid sequence that is at least 85% identical to the amino acid sequence of SEQ ID NO: 1.

The present invention further relates to a method for producing a poly(3-hydroxyalkanoate), the method including: culturing a microorganism having a poly(3-hydroxyalkanoate)-producing ability in the presence of a carbon source, wherein the microorganism is the transformed microorganism as defined above, and the carbon source contains an oil containing unsaturated fatty acids as constituents or contains free unsaturated fatty acids.

### Advantageous Effects of Invention

One aspect of the present invention can provide a method for culturing a microorganism, the method being adapted to achieve a high cell proliferation rate despite the use of an oil containing unsaturated fatty acids as constituents or of a free unsaturated fatty acid as a carbon source.

Other aspects of the present invention can provide a transformed microorganism and a method for producing a PHA, the transformed microorganism and the method being adapted to achieve a high cell proliferation rate and a high PHA production rate despite the use of an oil containing unsaturated fatty acids as constituents or of a free unsaturated fatty acid as a carbon source.

### Description of Embodiments

Hereinafter, specific embodiments of the present invention will be described in detail. The present invention is not limited to the embodiments described below.

One embodiment relates to a method for culturing a microorganism, the method including culturing the microorganism in the presence of a carbon source. The microorganism is a transformed microorganism having an introduced foreign gene encoding a protein having an amino acid sequence of SEQ ID NO: 1 or an introduced foreign gene encoding a protein having an amino acid sequence that is at least 85% identical to the amino acid sequence of SEQ ID NO: 1.

### (Microorganism)

The transformed microorganism may be any microorganism that can assimilate oils or free fatty acids. The host of the transformed microorganism may be a microorganism isolated from a natural source, a mutant strain resulting from an artificial mutation, or a transformed strain resulting from a genetic manipulation using a genetic engineering technique. Examples of hosts preferred for use include, but are not limited to: bacteria belonging to the genus *Ralstonia*, the genus *Cupriavidus,* the genus *Wautersia*, the genus *Aeromonas*, the genus *Escherichia*, the genus *Alcaligenes*, the genus *Pseudomonas,* the genus *Bacillus,* the genus *Azotobacter*, the genus *Nocardia*, the genus *Sphingomonas,* or the genus *Comamonas;* and yeasts belonging to the genus *Saccharomyces*, the genus *Yarrowia,* or the genus *Candida.* The host used may be one that intrinsically has a fadH gene described below or may be one that is intrinsically devoid of the fadH gene.

### (fadH Gene)

In the present embodiment, the foreign gene introduced into the host is a fadH gene. The fadH gene encodes 2,4-dienoyl-CoA reductase. The 2,4-dienoyl-CoA reductase is an enzyme (EC 1.3.1.34) essential for beta-oxidation of unsaturated fatty acids having a double bond on an even-numbered carbon atom. In the beta-oxidation, the 2,4-dienoyl-CoA reductase catalyzes a reaction in which 2,4-dienoyl-CoA is reduced in an NADPH-dependent manner to give 3-trans-enoyl-CoA or 2-trans-enoyl-CoA.

Specifically, the fadH gene is preferably a gene encoding a protein having the amino acid sequence of SEQ ID NO: 1 or a gene encoding a protein having an amino acid sequence that is at least 85% identical to the amino acid sequence of SEQ ID NO: 1. The gene encoding a protein having the amino acid sequence of SEQ ID NO: 1 is a fadH gene obtained from *Escherichia coli.*

The protein having an amino acid sequence that is at least 85% identical to the amino acid sequence of SEQ ID NO: 1 is preferably a protein having 2,4-dienoyl-CoA reductase activity and having an amino acid sequence corresponding to the amino acid sequence of SEQ ID NO: 1 in which one or more amino acids have been replaced, deleted, inserted, or added. The sequence identity is preferably 90% or more, more preferably 95% or more, even more preferably 97% or more, and particularly preferably 99% or more.

The replacement, deletion, insertion, or addition of one or more amino acids is preferably a conservative mutation after which the protein function is kept intact and more preferably a conservative replacement. Examples of the conservative replacement include replacement between aromatic amino acids, replacement between hydrophobic amino acids, replacement between polar amino acids, replacement between basic amino acids, and replacement between amino acids having hydroxy groups. The replacement, deletion, insertion, or addition of one or more amino acids may be an artificial mutation or a natural mutation attributed to interindividual variation or species difference of the living organisms from which genes are derived.

The term "foreign gene" means that the species of the living organism from which the gene is derived differs from the species of the host of the transformed microorganism. One foreign gene may be introduced, or two or more foreign genes may be introduced.

The presence of a foreign fadH gene in the transformed microorganism can enhance the 2,4-dienoyl-CoA reductase activity. This can increase the rate of assimilation of unsaturated fatty acids by the transformed microorganism. In particular, in the early stage of culture, the proliferation of the microorganism is favored over substance accumulation, and the cell proliferation becomes more efficient as the assimilation rate of a fatty acid used as a carbon source increases. The efficient assimilation of unsaturated fatty acids in the early stage of cultivation is inferred to increase the cell proliferation rate when an oil containing unsaturated fatty acids as constituents or a free unsaturated fatty acid is used as a carbon source.

An example has been reported in which enhancement of endogenous fadH gene expression in an L-amino acid-producing bacterium belonging to the family *Enterobacteriaceae* is able to increase the assimilation rate of unsaturated fatty acids (see Patent Literature 2). However, since the components constituting microbial cells are very different from host to host, the metabolism and cell proliferation rate greatly vary depending on the host microorganism. It is therefore inferred that it is highly uncertain whether the foreign gene introduced into a host serves a desired function in the host.

The method for introducing a fadH gene into a host is not limited to a particular technique. Examples of the introduction method include: a method in which the target gene is directly inserted onto a chromosome of the host or a gene on the chromosome is replaced by the target gene; a method in which the target gene is directly inserted onto a megaplasmid possessed by the host or a gene on the megaplasmid is replaced by the target gene; and a method in which the target gene is placed on a vector such as a plasmid, phage, or phagemid and the vector harboring the gene is introduced into the host. Two or more of these methods may be used in combination.

In view of the stability of the introduced gene, preferred is the method in which the target gene is directly inserted onto a chromosome or a megaplasmid of the host or a gene on the chromosome or the megaplasmid is replaced by the target gene. More preferred is the method in which the target gene is directly inserted onto a chromosome of the host or a gene on the chromosome is replaced by the target gene. To ensure reliable expression of the introduced gene, it is preferable to introduce the target gene in such a manner that the target gene is located downstream of a "gene expression regulatory sequence" inherently possessed by the host or downstream of a foreign "gene expression regulatory sequence". The "gene expression regulatory sequence" may be a DNA sequence containing a base sequence that controls the level of transcription of the gene (e.g. promotor sequence) and/or a base sequence that regulates the level of translation of a messenger RNA transcribed from the gene (e.g. Shine-Dalgamo sequence). The "gene expression regulatory sequence" used may be any naturally occurring base sequence or an artificially constructed or altered base sequence.

The foreign gene introduction can be accomplished by a method known to those skilled in the art. Typical methods that can be used include: a method using a transposon and the mechanism of homologous recombination (Ohman et al., J. Bacteriol., 162:1068-1074 (1985)); a method based on site-specific integration caused by the mechanism of homologous recombination and on loss due to second homologous recombination (Noti et al., Methods Enzymol., 154:197-217 (1987)); a method in which a sacB gene derived from *Bacillus subtilis* is allowed to coexist and in which a microorganism strain having lost a gene due to second homologous recombination is easily isolated as a sucrose-resistant strain (Schweizer, Mol. Microbiol., 6:1195-1204 (1992) or Lenz et al., J. Bacteriol., 176:4385-4393 (1994)); and a method in which the foreign gene is introduced using a plasmid vector.

The foreign gene introduction into a microorganism by means of a plasmid vector can be accomplished by any commonly used method such as electroporation or calcium transformation. The plasmid vector may be prepared by conjugating a DNA fragment having the base sequences of the foreign gene to a plasmid vector such as pCUP2.

### (PHA Synthase Gene)

The transformed microorganism in the present embodiment may be a microorganism that has a gene encoding a PHA synthase and that has a PHA-producing ability. The use of a microorganism having a PHA synthase gene in the present embodiment leads to an increased cell proliferation rate and provides an increasing effect on the PHA production rate. The PHA synthase gene may be one intrinsically possessed by the transformed microorganism or may be a foreign gene.

The microorganism having the PHA synthase gene is not limited to a particular type, and examples of such a microorganism include: microorganisms belonging to the genus *Cupriavidus* such as *Cupriavidus necator*; microorganisms belonging to the genus *Alcaligenes* such as *Alcaligenes latus*; microorganisms belonging to the genus *Pseudomonas* such as *Pseudomonas putida, Pseudomonas fluorescens*, *Pseudomonas aeruginosa, Pseudomonas resinovorans,* and *Pseudomonas oleovorans;* microorganisms belonging to the genus *Bacillus* such as *Bacillus megaterium;* microorganisms belonging to the genus *Azotobacter;* microorganisms belonging to the genus *Nocardia;* microorganisms belonging to the genus *Aeromonas* such as *Aeromonas caviae* and *Aeromonas hydrophila);* microorganisms belonging to the genus *Ralstonia;* microorganisms belonging to the genus *Wautersia;* and microorganisms belonging to the genus Comamonas (Microbiological Reviews, pp. 450-472, 1990).

The transformed microorganism having an introduced foreign gene encoding a PHA synthase may be any of the microorganisms mentioned above or may be a gram-negative bacterium such as that belonging to the genus *Escherichia,* a gram-positive bacterium such as that belonging to the genus *Bacillus,* or a yeast such as that belonging to the genus *Saccharomyces,* the genus *Yarrowia,* or the genus *Candida.*

The microorganism having a PHA synthase gene is preferably a bacterium in order to accumulate a large amount of PHA. A bacterium belonging to the genus *Cupriavidus* is more preferred, and *Cupriavidus necator* is particularly preferred.

The PHA synthase gene is not limited to a particular type, and examples of the PHA synthase gene include: a gene derived from *Aeromonas caviae, Aeromonas hydrophila, Pseudomonas* SP 61-3, or *Cupriavidus necator* and encoding a PHA synthase; a gene encoding a chimera PHA synthase combining two or more such PHA synthases; and a gene encoding a protein having an amino acid sequence that is at least 85% identical to the amino acid sequence of the PHA synthase. The sequence identity is preferably 90% or more, more preferably 95% or more, even more preferably 97% or more, and particularly preferably 99% or more. One PHA synthase gene may be introduced, or two or more PHA synthase genes may be introduced. When two or more PHA synthase genes are introduced, the PHA synthase genes may be identical or different genes.

The PHA in the present disclosure is not limited to a particular type and may be any poly(3-hydroxyalkanoate) that can be microbially produced. Preferred examples of the PHA include: a homopolymer of one monomer selected from 3-hydroxyalkanoic acids having 4 to 16 carbon atoms; a copolymer of at least one monomer selected from 3-hydroxyalkanoic acids having 4 to 16 carbon atoms and another hydroxyalkanoic acid (e.g., a 4-hydroxyalkanoic acid having 4 to 16 carbon atoms or lactic acid); and a copolymer of two or more monomers selected from 3-hydroxyalkanoic acids having 4 to 16 carbon atoms. Specific examples of the PHA include, but are not limited to: P(3HB) which is a homopolymer of 3-hydroxybutyric acid (abbreviated as "3HB"); P(3HB-co-3HV) (abbreviated as "PHBV") which is a copolymer of 3HB and 3-hydroxyvaleric acid (abbreviated as "3HV"); P(3HB-co-3HH) (abbreviated as "PHBH") which is a copolymer of 3HB and 3-hydroxyhexanoic acid (abbreviated as "3HH"); P(3HB-co-4HB) which is a copolymer of 3HB and 4-hydroxybutyric acid (abbreviated as "4HB"); and a PHA containing lactic acid (abbreviated as "LA") as a constituent component (an example of this PHA is P(LA-co-3HB) which is a copolymer of 3HB and LA).

The PHA is preferably a PHA containing at least 3-hydroxybutyrate units in terms of using the polymer in a wide range of applications. The PHA is more preferably a homopolymer of 3-hydroxybutyrate units or a copolymer of 3-hydroxybutyrate units and other hydroxyalkanoate units. Among the copolymers mentioned above, PHBV and PHBH are more preferred, and PHBH is particularly preferred.

The type of the PHA to be produced can be chosen as appropriate depending on the type of the PHA synthase gene possessed by or introduced into the microorganism used, the type of the metabolic gene involved in the PHA synthesis, the culture conditions, and other factors.

### (Carbon Source)

In the present embodiment, the transformed microorganism described above can be cultured in the presence of a carbon source. Preferably, the carbon source contains an oil containing unsaturated fatty acids as constituents or contains a free unsaturated fatty acid. The oil or the free unsaturated fatty acid may be used alone, or they may be used in combination. Only one type of the oil or a mixture of multiple oils may be used. Only one type of a free unsaturated fatty acid or a mixture of multiple free unsaturated fatty acids may be used. The oil or the free unsaturated fatty acids may be used in combination with other carbon sources (such as sugars and saturated fatty acids).

The unsaturated fatty acids contained as constituents in the oil and the free unsaturated fatty acids are not limited to a particular type and may be any fatty acids having one or more unsaturated carbon-carbon bonds. Examples of the unsaturated fatty acids and the free unsaturated fatty acids include: monounsaturated fatty acids such as crotonic acid, myristoleic acid, palmitoleic acid, sapienic acid, oleic acid, elaidic acid, vaccenic acid, gadoleic acid, eicosenoic acid, erucic acid, and nervonic acid; diunsaturated fatty acids such as linoleic acid, eicosadienoic acid, and docosadienoic acid; and triunsaturated fatty acids such as linolenic acid, pinolenic acid, eleostearic acid, mead acid, dihomo-γ-linolenic acid, and eicosatrienoic acid. Among these, the diunsaturated fatty acids are preferred. Linoleic acid and linolenic acid are more preferred, and linoleic acid is particularly preferred.

In terms of achieving a higher cell proliferation rate, the total content (wt%) of the diunsaturated fatty acids is preferably 1 % or more, more preferably 5% or more, and even more preferably 10% or more in the total constituent fatty acids of the oil or in the total free fatty acids.

The oil contains a triglyceride which is an ester compound of fatty acids and glycerin. The fatty acids in the triglyceride (referred to as "constituent fatty acids") include an unsaturated fatty acid and may include a saturated fatty acid in addition to the unsaturated fatty acid. The oil used is not limited to a particular type and may be an animal oil, a vegetable oil, a mixture of animal and vegetable oils, a transesterified oil, or a fractionated oil. Specific examples of the vegetable oil include rapeseed oil, sunflower oil, soybean oil, olive oil, corn oil, palm oil, palm kernel oil, cottonseed oil, sesame oil, nut oil, Jatropha oil, and rice oil. Specific examples of the animal oil include lard. One of the above-mentioned oils may be used alone, or a mixture of two or more thereof may be used.

To enhance the effect achieved by the present invention, the carbon source preferably has a high unsaturated fatty acid content, namely a high iodine value. Specifically, the iodine value of the carbon source is preferably 1 or more, more preferably 5 or more, even more preferably 8 or more, still even more preferably 50 or more, particularly preferably 80 or more, and most preferably 100 or more. The upper limit of the iodine value is not limited to a particular value. For example, the iodine value may be 200 or less or may be 180 or less. The iodine value can be adjusted by selecting the type of the oil or free fatty acid used, selecting the combination of two or more types of oils or free fatty acids, or selecting the proportions of the oils or free fatty acids.

The iodine value is an index of the amount of unsaturated bonds contained in an oil or a free fatty acid and represents the grams of iodine that can be added to 100 g of the oil or the free fatty acid. A higher iodine value indicates a greater number of double bonds contained in the oil or the free fatty acid and serves as evidence of a higher degree of unsaturation of the oil or the free fatty acid.

In the present embodiment, the transformed microorganism having an introduced fadH gene is cultured in the presence of an oil or a free fatty acid with a relatively high iodine value (high-iodine-value oil or free fatty acid) as a carbon source. The cell proliferation rate can be improved compared to the case where an equivalent transformed microorganism is cultured under the same conditions except the introduction of fadH gene. When the transformed microorganism has a PHA synthase gene, not only the cell proliferation rate but also the PHA production rate can be increased. Thus, in the present embodiment, a high-iodine-value oil or free fatty acid can be effectively used as a carbon source for the microorganism.

The way of adding the carbon source to a culture medium may be one-time addition or consecutive addition and is preferably consecutive addition. That is, it is preferable to cultivate the transformed microorganism while consecutively adding the carbon source to the culture medium and dispersing it. The phrase "consecutive addition" or "consecutively adding" as used herein is intended to include adding the carbon source continuously without any interruption and adding the carbon source intermittently several times at intervals.

### (Culture)

The culture medium used in the cultivation of the transformed microorganism may be any liquid culture medium containing nutrient sources conducive to the growth and proliferation of the microorganism. Preferably, the transformed microorganism is mixed with a liquid containing the carbon source as described above, a nitrogen source, an inorganic salt, and another organic nutrient source other than the carbon source and is dispersed in the liquid by stirring, shaking, or any other means.

Examples of the nitrogen source include ammonium salts such as ammonia, ammonium chloride, ammonium sulfate, and ammonium phosphate and further include peptone, meat extract, and yeast extract. Examples of the inorganic salt include potassium dihydrogen phosphate, disodium hydrogen phosphate, magnesium phosphate, magnesium sulfate, and sodium chloride. Examples of the other organic nutrient source include amino acids such as glycine, alanine, serine, threonine, and proline; and vitamins such as vitamin B1, vitamin B12, and vitamin C.

The culture medium containing the nutrient sources as described above, the carbon source, and the transformed microorganism are dispersed in a vessel to obtain a culture fluid. The culture conditions may be set as per common microbial culture, except for the carbon source described above and the way of adding the carbon source. There is no particular limitation on the culture scale, the aeration/stirring conditions, the culture temperature, the pH during culture, the culture time, etc.

### (Collection of PHA)

When the transformed microorganism has a PHA synthase gene, the microorganism is cultured for a suitable time to allow the microorganism to accumulate a PHA in the cells, and then the PHA is collected from the cells using a known technique. The PHA collection is not limited to a particular technique and can be accomplished, for example, as follows. After the end of the culture, the microbial cells are separated from the culture fluid by means such as a centrifuge, and the separated microbial cells are washed with a liquid such as distilled water or methanol and then dried. The PHA is extracted from the dried microbial cells using an organic solvent such as chloroform. The cellular components are removed from the PHA-containing solution through a process such as filtration, and a poor solvent such as methanol or hexane is added to the filtrate to precipitate the PHA. The supernatant fluid is removed through filtration or centrifugation, and the PHA is dried and collected.

In another example, the microbial cells are separated from the culture fluid by means such as a centrifuge, and the separated microbial cells are washed with a liquid such as distilled water or methanol. Subsequently, the washed sample is mixed with a solution of sodium lauryl sulfate (SDS), and the mixture is subjected to ultrasonic disruption to break the cell membranes. The cellular components and the PHA are then separated by means such as a centrifuge, and the PHA is dried and collected.

In the following items, preferred aspects of the present disclosure are listed. The present invention is not limited to the following items.

### [Item 1]

A method for culturing a microorganism, the method including:
culturing the microorganism in the presence of a carbon source, wherein
the microorganism is a transformed microorganism having an introduced foreign gene encoding a protein having an amino acid sequence of SEQ ID NO: 1 or an introduced foreign gene encoding a protein having an amino acid sequence that is at least 85% identical to the amino acid sequence of SEQ ID NO: 1, and
the carbon source contains an oil containing an unsaturated fatty acid as a constituent fatty acid or contains a free unsaturated fatty acid.

### [Item 2]

The method according to item 1, wherein an iodine value of the carbon source is 8 or more.

### [Item 3]

A transformed microorganism having:
a gene encoding a poly(3-hydroxyalkanoate) synthase; and
an introduced foreign gene encoding a protein having an amino acid sequence of SEQ ID NO: 1 or an introduced foreign gene encoding a protein having an amino acid sequence that is at least 85% identical to the amino acid sequence of SEQ ID NO: 1.

### [Item 4]

The transformed microorganism according to item 3, wherein a host of the transformed microorganism belongs to the genus *Cupriavidus.*

### [Item 5]

The transformed microorganism according to item 4, wherein the host is *Cupriavidus necator.*

### [Item 6]

A method for producing a poly(3-hydroxyalkanoate), the method including:
culturing a microorganism having a poly(3-hydroxyalkanoate)-producing ability in the presence of a carbon source, wherein
the microorganism is the transformed microorganism according to any one of items 3 to 5, and
the carbon source contains an oil containing unsaturated fatty acids as constituents or contains free unsaturated fatty acids.

### [Item 7]

The method according to item 6, wherein the poly(3-hydroxyalkanoate) contains at least 3-hydroxybutyrate units.

### [Item 8]

The method according to item 7, wherein the poly(3-hydroxyalkanoate) includes a homopolymer of 3-hydroxybutyrate units or a copolymer of 3-hydroxybutyrate units and other hydroxyalkanoate units.

### [Item 9]

The method according to item 8, wherein the other hydroxyalkanoate units are 3-hydroxyhexanoate units.

### Examples

Hereinafter, the present invention will be described in more detail using examples. The present invention is not limited to the examples given below.

In the examples and comparative examples described below, oils having different iodine values were used. The oils are listed in Table 1.

**[Table 1]**

| Constituent fatty acids (Unit: wt%) | Oil 1 | Oil 2 | Oil 3 | Oil 4 |
|---|---|---|---|---|
| Octanoic acid (8) | 7 | 0 | 0 | 0 |
| Capric acid (10) | 5.6 | 0 | 0 | 0 |
| Lauric acid (12) | 45.1 | 0 | 0 | 0 |
| Myristic acid (14) | 18.3 | 1 | 0.2 | 0 |
| Myristoleic acid (14:1) | 0 | 0 | 0 | 0 |
| Palmitic acid (16) | 13 | 39.6 | 10.5 | 10.5 |
| Palmitoleic acid (16:1) | 0 | 0.2 | 0.2 | 0 |
| Stearic acid (18) | 3 | 4.4 | 2.4 | 3.9 |
| Oleic acid (18:1) | 5.5 | 42.7 | 53.7 | 24.9 |
| Linoleic acid (18:2) | 2.2 | 11.2 | 24.9 | 52.4 |
| Linolenic acid (18:3) | 0.1 | 0.2 | 6.2 | 7.2 |
| (20-24) | 0 | 0.2 | 1.9 | 1 |
| Unsaturated fatty acid content (wt%) | 8 | 54 | 85 | 85 |
| Iodine value | 8 | 57 | 108 | 131 |

### (How to Measure Iodine Value of Carbon Source)

The iodine value of each carbon source was determined by a redox titration as specified in the JIS standards. In general, Wijs method using iodine monochloride or Hanus method using iodine bromide is widely used to promote the iodine addition reaction.

### (How to Measure Turbidity (OD600))

During cultivation, 1 ml of the culture fluid was collected, and the cellular components were separated from the liquid by centrifugation. The separated cellular components were washed with ethanol and pure water. A suspension sample containing the cellular components and diluted with pure water was subjected to measurement using a spectrophotometer. The suspension sample was irradiated with light having a wavelength of 600 nm by means of the spectrophotometer to cause light scattering, based on which the turbidity (OD600) of the suspension sample was determined.

The turbidity is a measure of the cell concentration in the culture fluid. A greater turbidity indicates a higher cell concentration and a greater number of proliferated cells.

### (Purification)

After the end of cultivation, the microbial cells were collected by centrifugation, washed with ethanol, and then dried under vacuum to obtain dry microbial cells.

To 1 g of the obtained dry microbial cells was added 100 ml of chloroform, and the microbial cells in chloroform were stirred for a whole day at room temperature to extract a PHA from the microbial cells. The microbial cell residues were removed by filtration, and the filtrate was concentrated using an evaporator to a total volume of 30 ml. To the concentrate was slowly added 90 ml of hexane, and the mixture was gently stirred for 1 hour. The PHA precipitated was collected by filtration and dried under vacuum at 60°C for 3 hours to obtain a dry PHA. The weight of the obtained dry PHA was measured, and the measured weight was used to calculate the PHA productivity in the way described below.

### (How to Calculate PHA Productivity)

The PHA productivity (%) is the proportion of the weight (g) of the PHA obtained from the culture fluid 72 hours after the start of cultivation in Example to the weight (g) of the PHA obtained from the culture fluid 72 hours after the start of cultivation in Comparative Example and was calculated using the equation shown below. This proportion was calculated for Example and Comparative Example in which the same oil was used as a carbon source. PHA productivity (%) = [weight (g) of PHA obtained in Example] / [weight (g) of PHA obtained in Comparative Example] × 100

### (Production Example 1) Preparation of pCUP2-trp-FadH/KNK005 or pCUP2-trp-FadM Cupriavidus necator H16

The overall genetic manipulation can be carried out according to Molecular Cloning (Cold Spring Harbor Laboratory Press (1989 or 2001)). The enzymes, cloning hosts, and other materials used in the genetic manipulation can be purchased from market suppliers and used according to the instructions given by the suppliers. The enzymes are not limited to particular type and may be any enzymes that can be used for genetic manipulations.

First, a plasmid vector was prepared. The preparation was done as follows.

PCR using synthetic oligo DNA was carried out to obtain a DNA fragment (SEQ ID NO: 2) having a Shine-Dalgarno sequence which is a ribosomal binding site, a trp promoter, and a base sequence encoding an amino acid sequence (SEQ ID NO: 1) of fadH derived from *Escherichia coli* K-12. The obtained DNA fragment was digested with restriction enzymes EcoRI and Spel. The resulting DNA fragment was conjugated to a plasmid vector pCUP2 which is described in WO 2007/049716 and which was cleaved with restriction enzymes MunI and SpeI, and thus a plasmid vector pCUP2-trp-fadH was obtained.

Subsequently, the plasmid vector pCUP2-trp-fadH was introduced into KNK005 or *Cupriavidus necator* H16 to obtain a transformed strain pCUP2-trp-fadH/KNK005 or pCUP2-trp-fadH/*Cupriavidus necator* H16.

The introduction of the plasmid vector into cells was carried out by electroporation as described below. The gene introduction device used was Gene Pulser manufactured by Bio-Rad Laboratories, Inc., and the cuvette used was a 0.2-cm-gap cuvette also manufactured by Bio-Rad Laboratories, Inc. The cuvette was charged with 400 µl of competent cells and 20 µl of expression vector and set on the pulse device, by which electric pulse was applied to the contents of the cuvette at a capacitance of 25 µF, a voltage of 1.5 kV, and a resistance value of 800 Ω. After the pulse application, the cell-containing fluid in the cuvette was subjected to shake culture in Nutrient Broth (manufactured by Difco Laboratories) at 30°C for 3 hours and then to culture on a selection plate (Nutrient Agar manufactured by Difco Laboratories, containing 100 mg/L kanamycin) at 30°C for 2 days. The transformed strain pCUP2-trp-fadH/KNK005 or pCUP2-trp-fadH/*Cupriavidus necator* H16 grown was collected.

### (Comparative Examples 1 to 4)

KNK-005 (see U.S. Patent No. 7384766) was used as a PHA-producing microorganism and subjected to a series of culture procedures as described below.

### (Culture Process)

The strain was cultured as follows.

The seed culture medium was composed of 1% (w/v) Meat extract, 1% (w/v) Bacto Tryptone, 0.2% (w/v) Yeast extract, 0.9% (w/v) Na₂HPO₄•12H₂O, 0.15% (w/v) KH₂PO₄ (pH = 6.8), and 5 × 10⁻⁶% (w/v) kanamycin. The PHA production culture medium was composed of 1.1% (w/v) Na₂HPO₄•12H₂O, 0.19% (w/v) KH₂PO₄, 0.13% (w/v) (NH₄)₂SO₄, 0.1% (w/v) MgSO₄•7H₂O, and 0.1% (v/v) trace metal salt solution (solution of 1.6% (w/v) FeCl₃•6H₂O, 1% (w/v) CaCl₂•2H₂O, 0.02% (w/v) CoCl₂•6H₂O, 0.016% (w/v) CuSO₄•5H₂O, and 0.012% (w/v) NiCl₂•6H₂O in 0.1N hydrochloric acid).

### (Preculture)

The strain was inoculated into 10 ml of the seed culture medium and cultivated at a culture temperature of 30°C for 17 hours to prepare a preculture fluid.

### (Main Culture)

Next, 1.0% (v/v) of the preculture fluid was inoculated to a Sakaguchi flask containing 50 ml of the PHA production culture medium and shake cultivation was performed at a culture temperature of 30°C for 72 hours.

Oils having iodine values as listed in Table 1 were used as carbon sources for the cultivation procedures. Table 2 shows the turbidity (OD600) of the culture fluid as measured 24 hours, 48 hours, and 72 hours after the start of culture. The PHA weight as measured 72 hours after the start of culture in each of Comparative Examples is indicated as 100 wt%.

### (Examples 1 to 4)

The pCUP2-trp-fadH/KNK005 produced in Production Example 1 was used as a PHA-producing microorganism and subjected to a series of culture procedures which were the same as those in Comparative Examples 1 to 4 described above.

Table 2 shows the turbidity (OD600) of the culture fluid as measured 24 hours, 48 hours, and 72 hours after the start of culture. Table 2 further shows the PHA productivity calculated as the proportion of the PHA weight as measured 72 hours after the start of culture to the PHA weight as measured in Comparative Example where the same oil was used in the culture procedures.

The PHA produced by the microbial cells in Comparative Examples 1 to 4 and Examples 1 to 4 is PHBH.

**[Table 2]**

| | *fadH* gene introduction | Oil type | Iodine value of oil | Turbidity (OD600) | | | PHA productivity (%) |
|---|---|---|---|---|---|---|---|
| | | | | 24 hr | 48 hr | 72 hr | |
| Comp. 1 | Not performed | Oil 1 | 8 | 16.9 | 59.0 | 60.8 | 100 |
| Ex. 1 | Performed | Oil 1 | 8 | 18.8 | 60.0 | 62.0 | 106 |
| Comp. 2 | Not performed | Oil 2 | 57 | 14.3 | 58.0 | 61.4 | 100 |
| Ex. 2 | Performed | Oil 2 | 57 | 20.1 | 60.2 | 66.7 | 108 |
| Comp. 3 | Not performed | Oil 3 | 108 | 14.6 | 51.8 | 58.2 | 100 |
| Ex. 3 | Performed | Oil 3 | 108 | 27.9 | 54.2 | 62.3 | 114 |
| Comp. 4 | Not performed | Oil 4 | 131 | 13.8 | 49.8 | 54.3 | 100 |
| Ex. 4 | Performed | Oil 4 | 131 | 24.3 | 52.3 | 62.1 | 116 |

Table 2 reveals the following findings. Comparison of Comparative Example 1 and Example 1 where the same oil was used shows that the turbidity (OD600) of the culture fluid was generally higher in Example 1 than in Comparative Example 1, and in particular, the turbidity as measured at 24 hours was high in Example 1. This leads to the conclusion that the fadH gene introduction into the microorganism increased the cell proliferation rate.

It is also seen that Example 1 exhibited a higher PHA productivity than Comparative Example 1 and achieved an increase in PHA production rate.

The above discussion also applies to comparison of Example 2 and Comparative Example 2, comparison of Example 3 and Comparative Example 3, and comparison of Example 4 and Comparative Example 4.

The results of Examples 1 to 4 and Comparative Examples 1 to 4 further show that the higher iodine value of the oil led to the greater increment in turbidity and PHA productivity of Example compared to those of Comparative Example . This leads to the conclusion that the effect of the fadH gene introduction on the cell proliferation rate and the PHA production rate is enhanced with increasing iodine value of the oil.

### (Comparative Examples 5 to 8)

*Cupriavidus necator* H16 (Aaceli Flores-Sanchez., et al., IJBM., 164: 1600-1607 (2020)) was used as a PHA-producing microorganism and subjected to a series of cultivation procedures which were the same as those in Comparative Examples 1 to 4 and Examples 1 to 4.

Oils having iodine values as listed in Table 1 were used as carbon sources for the cultivation procedures. Table 3 shows the turbidity (OD600) of the culture fluid as measured 24 hours, 48 hours, and 72 hours after the start of cultivation. The PHA weight as measured 72 hours after the start of cultivation in each of Comparative Examples is indicated as 100 wt%.

### (Examples 5 to 8)

The pCUP2-trp-fadH/*Cupriavidus necator* H16 produced in Production Example 1 was used as a PHA-producing microorganism and subjected to a series of cultivation procedures which were the same as those in Comparative Examples 1 to 4 and Examples 1 to 4.

Table 3 shows the turbidity (OD600) of the culture fluid as measured 24 hours, 48 hours, and 72 hours after the start of cultivation. Table 3 further shows the PHA productivity calculated as the proportion of the PHA weight as measured 72 hours after the start of cultivation to the PHA weight as measured in Comparative Example where the same oil was used in the cultivation procedures.

The PHA produced by the microbial cells in Comparative Examples 5 to 8 and Examples 5 to 8 is P(3HB).

**[Table 3]**

| | *fadH* gene introduction | Oil type | Iodine value of oil | Turbidity (OD600) | | | PHA productivity (%) |
|---|---|---|---|---|---|---|---|
| | | | | 24 hr | 48 hr | 72 hr | |
| Comp. 5 | Not performed | Oil 1 | 8 | 14.2 | 52.2 | 61.5 | 100 |
| Ex. 5 | Performed | Oil 1 | 8 | 16.3 | 54.1 | 62.0 | 102 |
| Comp. 6 | Not performed | Oil 2 | 57 | 16.6 | 54.9 | 63.9 | 100 |
| Ex. 6 | Performed | Oil 2 | 57 | 21.3 | 59.9 | 65.0 | 105 |
| Comp. 7 | Not performed | Oil 3 | 108 | 13.3 | 49.9 | 57.7 | 100 |
| Ex. 7 | Performed | Oil 3 | 108 | 22.6 | 56.3 | 60.1 | 111 |
| Comp. 8 | Not performed | Oil 4 | 131 | 13.8 | 49.8 | 54.3 | 100 |
| Ex. 8 | Performed | Oil 4 | 131 | 16.9 | 53.3 | 60.2 | 116 |

Table 3 reveals the following findings. Comparison of Comparative Example 5 and Example 5 where the same oil was used shows that the turbidity (OD600) of the culture fluid was generally higher in Example 5 than in Comparative Example 5, and in particular, the turbidity as measured at 24 hours was high in Example 5. This leads to the conclusion that the fadH gene introduction into the microorganism increased the cell proliferation rate.

It is also seen that Example 5 exhibited a higher PHA productivity than Comparative Example 5 and achieved an increase in PHA production rate.

The above discussion also applies to comparison of Example 6 and Comparative Example 6, comparison of Example 7 and Comparative Example 7, and comparison of Example 8 and Comparative Example 8.

The results of Examples 5 to 8 and Comparative Examples 5 to 8 further show that the higher iodine value of the oil led to the greater increment in turbidity and PHA productivity of Example compared to those of Comparative Example. This leads to the conclusion that the effect of the fadH gene introduction on the cell proliferation rate and the PHA production rate is enhanced with increasing iodine value of the oil.

## Claims

1. A method for culturing a microorganism, the method comprising:
culturing the microorganism in the presence of a carbon source, wherein
the microorganism is a transformed microorganism comprising an introduced foreign gene encoding a protein comprising an amino acid sequence of SEQ ID NO: 1 or an introduced foreign gene encoding a protein comprising an amino acid sequence that is at least 85% identical to the amino acid sequence of SEQ ID NO: 1, and
the carbon source comprises an oil containing an unsaturated fatty acid as a constituent fatty acid or comprises a free unsaturated fatty acid.

2. The method according to claim 1, wherein an iodine value of the carbon source is 8 or more.

3. A transformed microorganism comprising:
a gene encoding a poly(3-hydroxyalkanoate) synthase; and
an introduced foreign gene encoding a protein comprising an amino acid sequence of SEQ ID NO: 1 or an introduced foreign gene encoding a protein comprising an amino acid sequence that is at least 85% identical to the amino acid sequence of SEQ ID NO: 1.

4. The transformed microorganism according to claim 3, wherein a host of the transformed microorganism belongs to the genus *Cupriavidus.*

5. The transformed microorganism according to claim 4, wherein the host is *Cupriavidus necator.*

6. A method for producing a poly(3-hydroxyalkanoate), the method comprising:
culturing a microorganism having a poly(3-hydroxyalkanoate)-producing ability in the presence of a carbon source, wherein
the microorganism is the transformed microorganism according to claim 3 or 4, and
the carbon source comprises an oil containing an unsaturated fatty acid as a constituent fatty acid or comprises a free unsaturated fatty acid.

7. The method according to claim 6, wherein the poly(3-hydroxyalkanoate) comprises at least 3-hydroxybutyrate units.

8. The method according to claim 7, wherein the poly(3-hydroxyalkanoate) comprises a homopolymer of 3-hydroxybutyrate units or a copolymer of 3-hydroxybutyrate units and other hydroxyalkanoate units.

9. The method according to claim 8, wherein the other hydroxyalkanoate units are 3-hydroxyhexanoate units.
